# EUROPEAN PATENT SPECIFICATION

(11) **EP 1 729 681 B1**
(45) Date of publication and mention of the grant of the patent: **25.04.2012**
(21) Application number: 05713922.2
(22) Date of filing: 22.02.2005
(51) Int. Cl.: A61F 2/90

(54) **PARTIALLY BIODEGRADABLE STENT**
TEILWEISE BIOLOGISCH ABBAUBARER STENT
STENT PARTIELLEMENT BIODEGRADABLE

(30) Priority: 31.03.2004 US 815115
(43) Date of publication of application: 13.12.2006
(73) Proprietor: Cook Medical Technologies LLC, Bloomington, IN 47404 (US)
(72) Inventor: SCHAEFFER, Darin, G., Bloomington, IN 47403 (US); PAUL, Ram, H., Bloomington, IN 47403 (US)
(74) Representative: Jehan, Robert
(86) International application number: PCT/US2005/005568
(87) International publication number: WO 2005/102220

(56) References cited:
- WO-A-00/62708
- WO-A-2004/045474
- WO-A-2004/052237
- US-A1- 2002 151 964

## Description

### BACKGROUND

This invention relates to a partially biodegradable radially expandable stent.

Surgical stents have long been known which can be surgically implanted into a body lumen, such as an artery, to reinforce, support, repair or otherwise enhance the performance of the lumen. For instance, in Interventional Cardiology it is often desirable to place a stent in the coronary artery at a location where the artery is narrowed or is susceptible to closure. The stent, once in place, reinforces that portion of the artery allowing normal blood flow to occur through the artery. One form of stent which is particularly desirable for implantation in arteries and other body lumens is a cylindrical stent which can be radially expanded from a first smaller diameter to a second larger diameter. Such radially expandable stents can be inserted into the artery by being located on a balloon and fed internally through a guiding catheter placed in the vasculature of the patient until the unexpanded stent is located where desired. The balloon or other expansion mechanism is then inflated which exerts a radial pressure outward on the stent causing the stent to expand radially to a larger diameter. Still other stents are self-expanding. Regardless of the expansion method, expandable stents exhibit sufficient rigidity after being expanded that they will remain expanded after the catheter has been removed.

Radially expandable stents come in a variety of different configurations to provide optimal performance in various different particular circumstances. For instance, the patents to Lau (U.S. Pat. Nos. 5,514,154, 5,421,955, and 5,242,399), Baracci (U.S. Pat. No. 5,531,741), Gaterud (U.S. Pat. No. 5,522,882), Gianturco (U.S. Pat. Nos. 5,507,771 and 5,314,444), Termin (U.S. Pat. No. 5,496,277), Lane (U.S. Pat. No. 5,494,029), Maeda (U.S. Pat. No. 5,507,767), Marin (U.S. Pat. No. 5,443,477), Khosravi (U.S. Pat. No. 5,441,515), Jessen (U.S. Pat. No. 5,425,739), Hickle (U.S. Pat. No. 5,139,480), Schatz (U.S. Pat. No. 5,195,984), Fordenbacher (U.S. Pat. No. 5,549,662) and Wiktor (U.S. Pat. No. 5,133,732), each include some form of radially expandable stent for implantation into a body lumen.

As the above listed patents demonstrate, a wide variety of stents are known in the prior art. Typically, stents are tube-shaped and flexible in their pre-expanded configuration. Stents often comprise a plurality of cylindrical, serpentine rings joined by connecting members. The connecting members for many of the prior art stents are flexible before the stent is expanded. When a stent is implanted (i.e., expanded) into a vessel, good scaffolding is desired initially to support the tissue and keep the vessel patent. Many of the prior art stents adequately support the tissue upon their deployment, and quite often, the connecting members become relatively rigid after expansion. However, because the connecting members are often relatively rigid after deployment, many of the prior art stents experience a problem with causing mechanical irritation to the tissue due to their lack of flexibility.
WO-00/62,708, which discloses all the features of the preamble of claim 1, discloses a multi-section stent which includes a connecting structure that allows the stent sections to move and flex relative to one another. For deployment and positioning, the connecting structure connects the multiple stent sections and holds the stent sections substantial stationary relative to one another. Following deployment, the connecting structure allows the multiple stent sections to move relative to one another. Movable stent sections enable flexure of the stent upon deployment within a body lumen. This flexing structure allows better conformance of the stent to the shape of the body lumen, and exerts less overall pressure against the lumen wall, reducing the potential for trauma. Upon deployment, the multiple stent sections may be completely detached from one another. Alternatively, the stent sections may remain partially connected in a manner that allows substantial independent movement. The connecting structure can be manufactured to separate upon deployment, for example, by tracking or degrading within the both lumen in which the stent is positioned.

What is needed is a stent which will remain relatively rigid for a certain amount of time after expansion within the vessel in order to provide structural support until the body endothelializes the stent, and which will then become more comfortable to avoid mechanical irritation to the body's tissue. The present invention satisfies these and other needs.

### SUMMARY

It is in an object of the invention to provide an expandable stent which after expansion with the body will become substantially rigid for a given time, and will then become increasingly flexible to avoid irritation to the body's tissue.

In one aspect, an expandable stent comprises a plurality of substantially cylindrical, serpentine ring structures. Each ring structure extends around a circumference of the stent and comprises at least one unit structure. The unit structure comprises a plurality of strut members and a plurality of bends which form a substantially zig-zag pattern. When the stent is in an unexpanded state, at least one connector member joins two of the ring structures. The connector member is biodegradable and adapted to biodegrade when the stent is in an expanded state so that the two ring structures become substantially disjoined.

In another aspect, a method of expanding a stent comprises providing an expandable stent. In an unexpanded state, the stent comprises a plurality of substantially cylindrical, serpentine ring structures. Each ring structure extends around a circumference of the stent, and at least one biodegradable connector member joins two of the ring structures. The stent is delivered in an unexpanded state to a desired deployment site within a mammalian body, where the stent is expanded. Finally, the connector member is biodegraded so that the two ring structures become substantially disjoined.

The expandable stent of the invention allows the stent to be expanded within the body and remain substantially rigid for a certain time, at which point the stent becomes increasingly conformable, due to the biodegradation of the connecting members, and thereby avoids mechanical irritation to the body's tissue.

The present invention, together with further objects and advantages, will be best understood by reference to the following detailed description taken in conjunction with the accompanying drawings.

### BRIEF DESCRIPTION OF SEVERAL VIEWS OF THE DRAWINGS

Figure 1 is a partial view of a prior art Zilver-stent in a pre-deployment configuration.

Figure 2 is a partial view of the stent of Figure 1 in a deployed configuration.

Figure 3 is a partial view of a V-flex stent having biodegradable connecting members in an expanded configuration prior to degradation of the connecting members.

Figure 4 is a partial view of the V-flex stent of Figure 3 in an expanded configuration after degradation of the connecting members.

Figure 5 is a partial view of a Z-stent having biodegradable connecting members.

Figure 6 is a cut open and unfolded view of a segment of interconnected spring members in a Z-stent device having biodegradable connecting members.

Figure 7 is an enlarged segment showing a biodegradable connecting member fastened to an arm section in a spring member of a Z-stent device.

Figure 8 is a view of one half of a Z-stent device shown without graft material.

Figures 9-11 are enlarged views of a biodegradable connecting member and spring members in a Z-stent device in an unloaded state, at an axial pressure load, and at an axial tensile load, respectively.

Figure 12 is a partial view of the Z-stent of Figure 6 in an expanded configuration after degradation of the connecting members.

Figure 13 is a partial view of an abdominal aorta aneurysm (AAA) device having biodegradable connecting members.

Figure 14 is a partial view of the AAA device of Figure 13 in an expanded configuration after degradation of the connecting members.

### DETAILED DESCRIPTION OF THE PREFERRED EMBODIMENTS

The following description of preferred embodiments of the invention provides examples of the present invention. The embodiments discussed herein are merely exemplary in nature, and are not intended to limit the scope of the invention in any manner. Rather, the description of these preferred embodiments serves to enable a person of ordinary skill in the art to make and use the present invention.

As shown in the drawings for purposes of illustration, the present invention is directed to a partially biodegradable stent. The invention is applicable to stents having different types of configurations as known in the art, such as a V-flex stent, a Zilver-stent, a Z-stent, and a Y-shaped (or AAA "abdominal aorta aneurysm" device) stent, and is applicable to different deployment systems known in the art, such as self-expanding or balloon expandable systems.

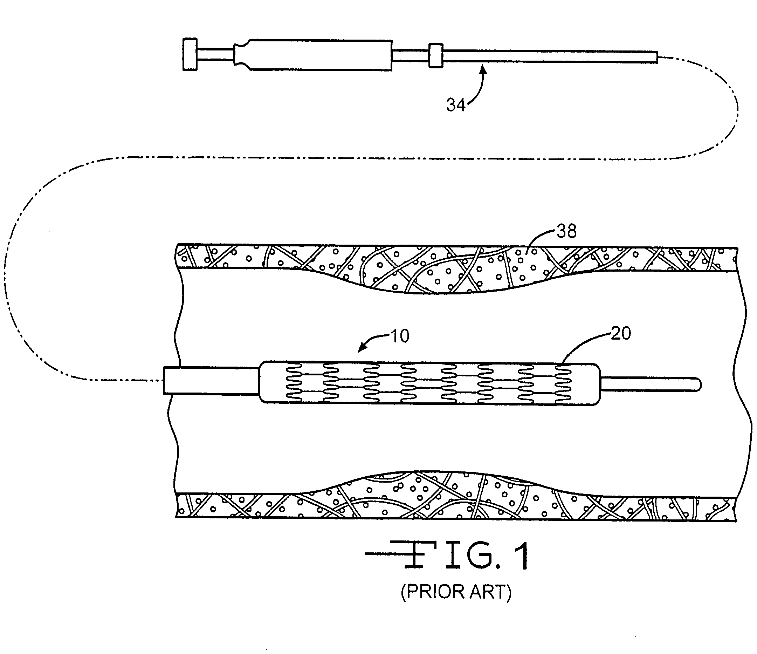

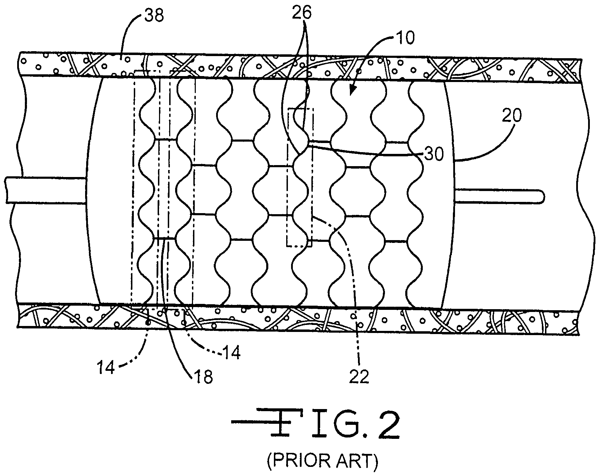

Fig. 1 shows a prior art Zilver-stent 10 mounted on a catheter device 34 in a pre-deployment (unexpanded) configuration. Before insertion into a patient's body, the stent 10 is placed over the expandable balloon 20 and crimped (collapsed, restrained) onto the balloon in order to form a reduced profile for insertion into the body through small diameter, tortuous-pathed arteries or other vessels or lumens within the body. In other prior art systems, a self-expanding stent may be utilized. Typically, the catheter device 34 is advanced through a patient's body in its unexpanded state by deploying a small guide wire (not shown) and advancing the catheter device over the guide wire to its final destination such as a partially blocked artery 38. The balloon 20 is then inflated by methods known in the art to expand the stent radially so that the stent 10 abuts against the walls of the artery 38 as shown in Fig. 2.

As seen in Fig. 2, the prior art stent 10 utilizes a plurality of ring structures 14 interconnected by a plurality of generally straight connector members 18. In other prior art, the connector members 18 may have flexible, curved portions, such as U, V, or W shaped portions, and the ring structures may be in varying configurations. Each ring structure 14 comprises a repeating pattern of unit structures 22. The unit structures 22 are made up of a repeated pattern of a plurality of struts 26 joined together by bends or apexes 30.

After deployment, the balloon 20 is deflated, and the catheter device is removed from the patient's body. The stent 10 remains in the expanded configuration in the artery to hold the artery open and prevent further blockage. In much of the prior art, the connector members 18, along with the stents themselves, remain relatively rigid after the stent is expanded in order to support the walls of the artery. Unfortunately, the lack of flexibility of these prior art stents after expansion often eventually leads to mechanical irritation of the tissue surrounding the stent.

The present invention solves this problem by providing a stent that, after expansion within a mammalian body, remains substantially rigid for a certain time, at which point the stent becomes increasingly conformable and thereby avoids mechanical irritation to the body's tissue. In order to accomplish this, the invention generally provides for biodegradable connector members which are substantially intact when the stent is in its unexpanded form, which remains substantially intact for some time after the stent is expanded within the body, and which after a given time substantially biodegrade. The remainder of the stent preferably retains its composition after expansion. In other embodiments, the remainder of the stent may contain biodegradable portions which also substantially biodegrade after a given time.

Preferably, the biodegradable connector members are made of one or more biodegradable polymers in varying combinations, such as polymers, copolymers, and block polymers. Some examples of such biodegradable (also bio-resorbable) polymers include poly-lactic acid, poly-glycolic acid, polyglycolides, polylactides, polycaprolactones, polyglycerol sebacate, polycarbonates e.g. tyrosine derived, polyethylene oxide, polybutylene terepthalate, polydioxanones, hybrids, composites, collagen matrices with growth modulators, proteoglycans, glycosaminoglycans, vacuum formed SIS (small intestinal submucosa), fibers, chitin, and dextran. Any of these biodegradable polymers may be used alone or in combination with these or other biodegradable polymers in varying compositions. The biodegradable connector members 18 may comprise a variety of shapes including, amongst others, a U shape, a V shape, a W shape, a generally curved shape, or may be substantially straight. The rest of the stent structure including the ring structures, is preferably made of one or more medically acceptable, i.e., biocompatible, materials which provide the desired longitudinal flexibility and radial strength. Some examples of suitable materials include shape memory alloys such as nitinol, stainless steel, 316 L stainless steel, cobalt chromium, nickel titanium, platinum, inconel, or any other material known in the art. As will be shown in differing embodiments below, the plurality of ring structures preferably form a substantially serpentine, cylindrical, and generally zig-zag shape extending around the circumference of the stent. Similarly, the unit structure preferably comprises a plurality of strut members and bends in a substantially zig-zag pattern. In other embodiments, the ring and unit structures may comprise other configurations, including a more linear, non-cylindrical configuration.

Preferably, for the embodiments discussed below, prior to expansion the stent forms a substantially flexible, integral, one-piece tubular shape having substantially flexible, biodegradable connector members. In other embodiments, the stent may comprise a variety of configurations such as a multiple piece tube or may have a non-tubular shape. After expansion and prior to the connector members biodegrading, the stent preferably becomes a relatively rigid, one-piece tube in order to provide the vessel or artery with good structural support until the body endothelializes the stent. In other embodiments, the stent may retain a non-tubular shape or multiple piece tubular shape. The biodegradable connector members are preferably substantially more rigid at this point relative to their pre-expanded state.

Preferably, for the embodiments discussed below, within the range of thirty (30) days to one-hundred-eighty (180) days after the stent has been expanded within the body, the connector members substantially biodegrade to leave a stent with substantially unconnected ring structures. However, longer or shorter degradation periods are also contemplated. The degradation rate is based in large part on what materials are utilized for the biodegradable connector members, in addition to the thickness of the connector members.
In some embodiments, the biodegradable connector members may comprise multiple layers of varying biodegradable polymers, each with differing degradation rates. Some layers may biodegrade quickly and other layers may undergo prolonged degradation. In other embodiments, the biodegradable connector members may comprise a single layer of a biodegradable polymer or a composite layer made up of a combination of varying biodegradable polymers. In these embodiments, the single layer may degrade at a substantially uniform rate.

In some embodiments, the ring structures may become completely disconnected. The structure of the remainder of the stent preferably retains its composition. At this point in time, the stent preferably becomes a modular, segmented stent comprising multiple disconnected tubular portions which allows the stent to become flexible and conformable so as to substantially avoid mechanical irritation to the body's tissue. In other embodiments, the stent may retain a non-tubular shape, or a substantially integral tubular shape.

In other embodiments, some of the remaining structure of the stent may also biodegrade. For instance, the ring structures may comprise a non-biodegradable base material, such as stainless steel, and the base material may be coated with one or more layers of biodegradable polymers in varying combinations and degradation rates. In these embodiments, within a given time after the stent is expanded (preferably within 30 to 180 days), the one or more layers of biodegradable polymers will biodegrade leaving only the non-biodegradable base material. At this time, the cross-section of the ring structures will be diminished leaving a more flexible stent structure. In other embodiments, the base structure may comprise a mixture of non-biodegradable materials and biodegradable polymers, with the biodegradable polymers biodegrading after a given time to leave only the non-biodegradable materials thereby allowing for more flexibility of the stent structure. In any of these embodiments, one or more drugs may be incorporated into one or more layers of the ring structures, such as anti-restenosis drugs, anti-inflammatory drugs, anti-thrombotic drugs, and growth factor or growth modulating drugs thereby inhibiting or accelerating tissue formation. These drugs may be mixed directly into one or more layers of the ring structures or may be enclosed within a cavity of the ring structures themselves for dispersion after biodegradation begins.

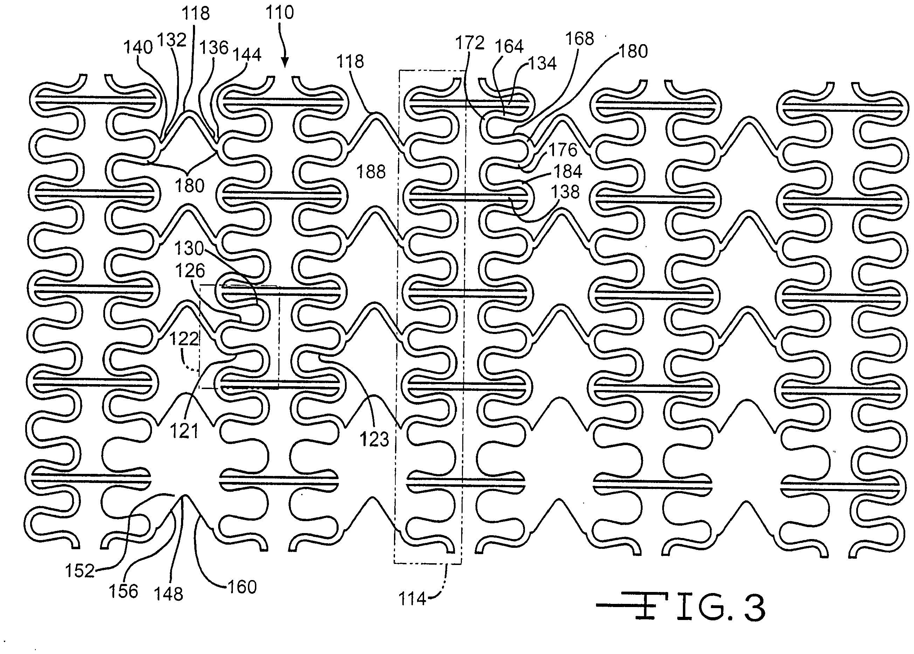

Figures 3-14 depict the invention. Fig. 3 illustrates a stent 110 in an expanded configuration shortly after deployment and prior to the biodegradable connector members degrading according to a preferred embodiment of the invention in which the substantially biodegradable connector members are utilized in a V-flex stent. Some representative examples of V-flex stents known in the art are disclosed in US patent number 5,843,175 and 5,868,782 to Global Therapeutics, Inc. and US patent number 6,042,606 to Cook, Inc. In the preferred embodiment, the stent 110 comprises a plurality of serpentine (zigzag or undulating) ring structures 114 interconnected by a plurality of biodegradable connector members 118. Each ring structure 114 is a substantially circular ring comprising an endless pattern of unit structures 122. The unit structure 122 represents a specific configuration of the wire members, including the struts 126 and bends 130, that comprise the structural component of the stent 110.

Preferably, as illustrated in Fig. 3, some adjacent unit structures 122 are connected together by first and second tie-bars 134 and 138. The repeated unit structure 122 of each ring structure 114 preferably comprises first and second struts 164 and 168 joined together by a first bend 172, a third strut 176 joined to the second strut 168 by a second bend 180, and a fourth strut 184 joined with the third strut 176 by a third bend 188. In other embodiments, the unit structure may be modified to include a varying number of struts and bends in differing configurations. The ring structures 114 depend on the configuration of the unit structures 122 and as a result may also comprise a variety of configurations in varying embodiments.

The struts and bends of the unit structure 122 preferably form repeated U or V shapes, but in other embodiments may be in varying configurations. The U or V shapes provide many advantages, such as being easily repeated around the circumference of the ring structures 114. Additionally, the U or V shapes facilitate the staggered arrangement of bends. Another advantage of using the U or V shapes is the availability of central attachment points for connecting members, as is more fully described below.

The ring structures 114 are interconnected to form the stent 110 by a plurality of biodegradable connector members 118. As illustrated in Fig. 3, each biodegradable connector member 118 joins first and second ring structures 114 and is preferably disposed between two adjacent unit structures 122. In other embodiments, each biodegradable connector member 118 may be disposed between adjacent unit structures in varying configurations, such as between W or Z shaped configurations.

Each biodegradable connector member 118 has a first end 132 and a second end 136. The first end 132 of each biodegradable connector member 118 is joined to a peak 140 of the second bend 180 of each unit structure 122 on the first ring structure 114. The second end 136 of each biodegradable connector member 118 is joined to a peak 144 of the second bend 180 of each adjacent unit structure 122 on the second ring structure 114. In other embodiments, varying configurations may be used to join the adjacent unit structures 122 of the ring structures 114 with the biodegradable connector members 118. For example, the biodegradable connector members 118 may join adjacent first bends 172 of adjacent unit structures 122 on separate ring structures 114, or may join adjacent third bends 188 of adjacent unit structures 122 on separate ring structures 114. As illustrated in Fig. 3, it is preferable that each unit structure 122 of each ring structure 114 is connected to two biodegradable connector members 118. In other embodiments, a varying number of biodegradable connector members 118 may be used to join each ring structure 114.

In addition to the first and second ends 132 and 136, each biodegradable connector member 118 also includes an undulating portion 148. The undulating portion 148 provides longitudinal flexibility to the stent 110. A variety of configurations can be used for the undulating portion 148, such as U, V, W, or Z shapes. The biodegradable connector segments 118 shown in Fig. 3 illustrate a preferred configuration for the undulating portions 148. In this preferred embodiment, each undulating portion 148 comprises an inverted "V" shape 152 made up of first and second legs 156 and 160. This configuration simplifies manufacture while providing the desired undulating portion 148 that confers axial flexibility onto the stent 110.

As illustrated in Fig. 3, it is preferable that the undulating portion 148 be positioned in the space 162 between each ring structure 114 without being overlapped by any of the tie-bars 134 and 138, struts 164, 168, 176, and 184, or bends 172, 180, and 188 of the adjacent unit structures 122. This allows the maximum possible flexibility of the undulating portion 148 by avoiding any potential abutment against the undulating portion 148. This configuration provides the desired axial flexibility while maintaining radial strength of the stent 110. Also, this configuration facilitates manufacturing due to the use of aligned portions. In other embodiments the undulating portion 148 may overlap the above-referenced members.

As depicted in Fig. 3, adjacent ring structures 114 are preferably radially aligned with each other. That is, adjacent ring structures 114 are preferably aligned such that axially adjacent unit structures 122 lie in complementary position relative to each other. Particularly preferable, all ring structures 114 of the stent 110 are radially aligned in this manner. However, other configurations may be used including staggered adjacent ring structures 114. As shown, it is preferable that the biodegradable connector members 118 joining adjacent ring structures 114 are in circumferential alignment relative to one another. This preferred arrangement arises due to the uniformity of the "V" shaped biodegradable connecting members 118 and unit structures 122, all arranged in alignment with one another. This arrangement maintains the desired axial flexibility while also contributing to the radial strength of the stent 110 and facilitating manufacturing. In other embodiments, the biodegradable connector members 118 joining adjacent ring structures 114 may be circumferentially displaced relative to one another.

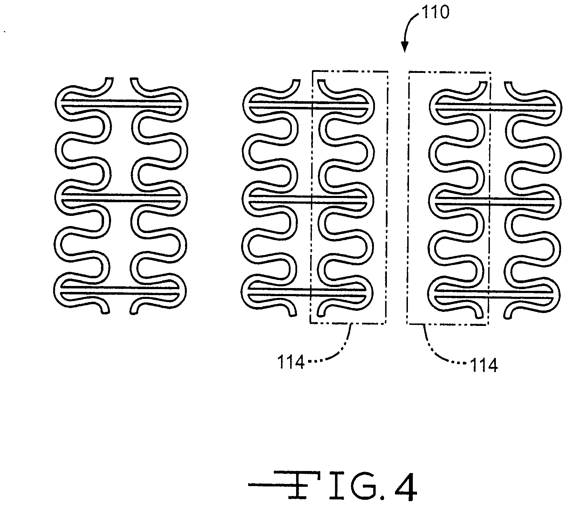

Fig. 4 illustrates the stent 110 of Fig. 3 in an expanded configuration within the range of thirty (30) to one-hundred-eighty (180) days after the stent 110 has been expanded within the body and the connector members 118 have substantially biodegraded to leave the stent 110 with a plurality of substantially unconnected ring structures 114. As shown, the structure of the remainder of the stent 110 preferably retains its composition although, as discussed previously, the remainder of the stent may also biodegrade. At this point in time, the stent 110 is a modular, segmented stent 110 comprising multiple disconnected ring structures 114 which are preferably generally tubular and which allow the stent 110 to become flexible and conformable so as to substantially avoid mechanical irritation to the body's tissue. In other embodiments, the stent may retain a non-tubular shape, or a substantially integral tubular shape.

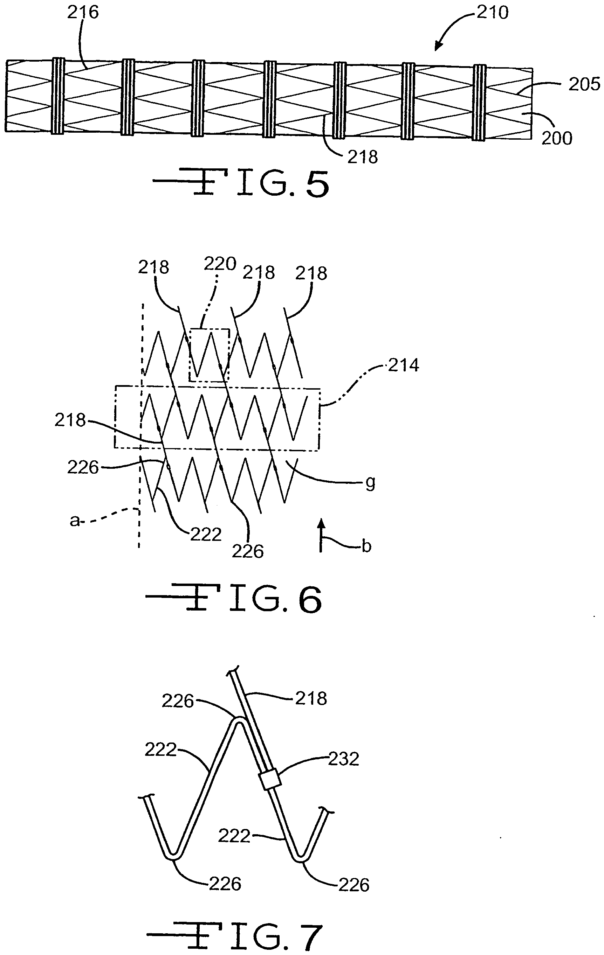

Fig. 5 illustrates a stent 210 in an expanded configuration shortly after deployment and prior to the biodegradable connector members 218 degrading according to a preferred embodiment of the invention in which the substantially biodegradable connector members 218 are utilized in a Z-stent. Some representative examples of Z-stents known in the art are disclosed in Australian patent number 729883 to William Cook Europe A/S.

As shown in Fig. 5, a Z-stent device 210 may be formed as a single tube, but a stent may also in other embodiments comprise a branched structure such as a Y-shaped structure with a relatively large tube branching into two smaller tubes. The stent device as a single tube can be used for treatment of a single-lumen vessel, such as to recreate the lumen of the vessel at a stenosis, for use as a vessel prosthesis at an aneurysm or a fistula, or at some other vessel anomaly. Under the invention, the stent device 210 comprises a graft 200 supported by tubular members 205, which are constructed from a plurality of cylindrical spring members 216 interconnected by biodegradable connecting members 218. A Z-stent device with a Y-shaped structure may be used to treat an abdominal aorta aneurysm.

The individual spring members 216 are Z-stents of a well-known design made of a wire of stainless steel, nitinol, a resilient plastics material, or a metallic material or composite which can exhibit elastic or superelastic properties. The wire is formed in a zig-zag configuration extending through a cylindrical surface, and the wire ends are joined together to make it endless, so that the cylindrical spring member is closed. Fig. 6 shows three spring members which are cut open along the line a and unfolded to a planar shape for the sake of clarity. The longitudinal direction of the tubular member is indicated by the arrow b. It should be noted that the different figures use the same reference numerals for details of the same type.

A spring member 216 includes a plurality of arm sections 222, each extending between two elbow sections 226. The arm sections are shown as approximately straight in their unloaded state, which is preferred to obtain high axial rigidity, but they may also be curved. The elbow sections are shown as simple bends in which the wire extends in a simple angle of a suitable radius of curvature. However, the elbow sections may have a more complex design in which the wire at the end of the arm section is formed, for example, as shown in Fig. 5 in EP-A1 0539237.

As shown in Fig. 6, the biodegradable connecting members 218 may extend obliquely in relation to the longitudinal direction b of the stent device 210. In the stent's expanded and unexpended states, the biodegradable connecting members 218 are seen to extend largely in parallel with the arm sections 222 and also mutually in parallel. The stent's ring structure is depicted by 214 and the stent's unit structure is depicted by 220. The embodiment shown in Fig. 6 is one preferred stent arrangement, although other arrangements may also be utilized. As shown in Fig. 6, a gap g follows a pair of interconnected arm sections, followed by another pair of interconnected arm sections. In the gap, the elbow sections are free to move closer to or further away from each other.

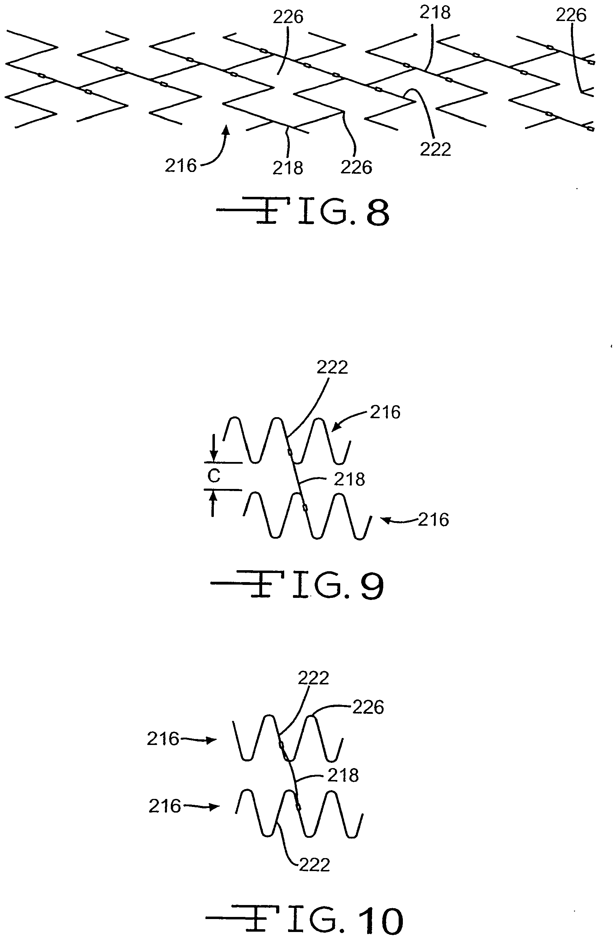

Fig. 8 shows an expanded view of the arrangement of Fig. 6. In the case of the advantageous locations of the biodegradable connecting members 218 shown in Fig. 8, between each of the arm sections having a mounted biodegradable connecting member 218 there is an arm section without any biodegradable connecting member. Thus both in the circumferential direction and in the longitudinal direction there is a free elbow section. With this open structure, the individual spring members can be resiliently deformed as indicated in Figs. 9-11. Fig. 9 shows the unloaded state in which the spring members 216 are maintained at the mutual distance c by means of the biodegradable connection member 218. The distance c can be freely adapted to the specific application by choice of a suitable length of the members 218.

At their ends the biodegradable connecting members 218 are firmly fastened to the associated arm sections 222. Fig. 7 shows that the fastening point 232 is at a distance from the elbow sections 226. Fastening is performed at both ends of the biodegradable connecting member. The connection may also be made in other areas, such as a distance away from the end sections of the biodegradable connecting member.

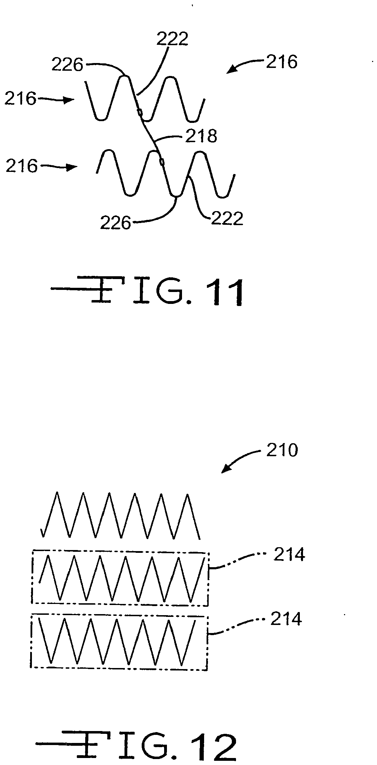

Fig. 12 illustrates the Z-stent 210 of Fig. 6 in an expanded configuration within the range of thirty (30) days to one-hundred-eighty (180) days after the stent 210 has been expanded within the body and the connector members 218 have substantially biodegraded to leave the stent 210 with a plurality of substantially unconnected ring structures 214. As shown, the structure of the remainder of the stent 210 preferably retains its composition although, as discussed previously, the remainder of the stent may also biodegrade. At this point in time, the stent 210 is a modular, segmented stent 210 comprising multiple disconnected ring structures 214 which are preferably generally tubular and which allow the stent 210 to become flexible and conformable so as to substantially avoid mechanical irritation to the body's tissue. In other embodiments, the stent may retain a non-tubular shape, or a substantially integral tubular shape.

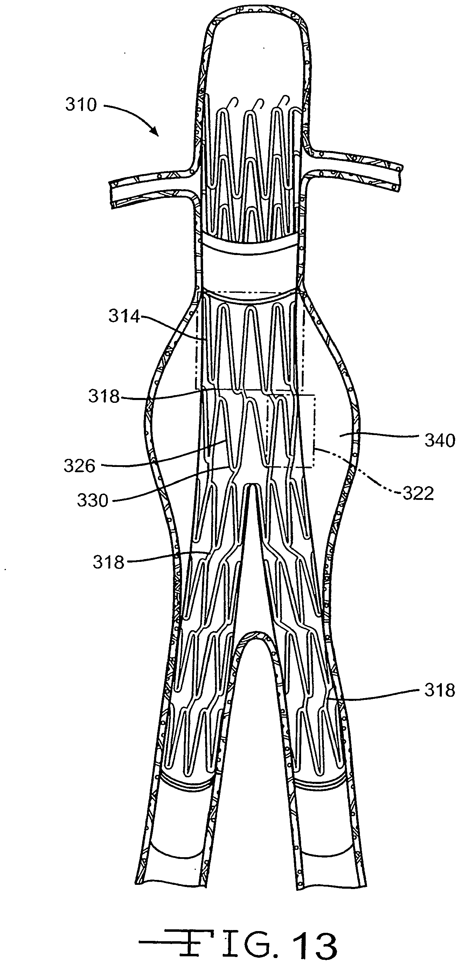

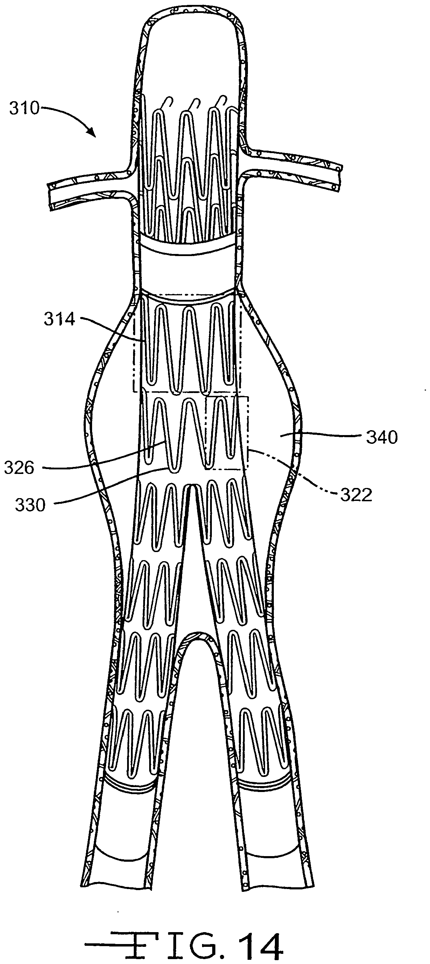

In yet another embodiment, as shown in Fig. 13, the present invention is utilized in a bifurcated stent device 310 with a Y-shaped structure which is used to treat an abdominal aorta aneurysm 340 (AAA device). Fig. 13 illustrates the AAA stent 310 in an expanded configuration shortly after deployment and prior to the biodegradable connector members 318 degrading. Such an AAA device is disclosed in PCT international publication number WO 98/53761 to William A. Cook. The stent 310 again comprises a plurality of cylindrical, ring structures 314, wherein each ring structure is comprised of one or more unit structures 322 formed of strut members 326 and bends 330 in a substantially zig-zagged pattern circumferentially around the stent. Fig. 14 illustrates the AAA stent 310 of Fig. 13 in an expanded configuration within the range of thirty (30) days to one-hundred-eighty (180) days after the stent 310 has been expanded within the body and the connector members 318 have substantially biodegraded to leave the stent 310 with substantially unconnected ring structures 314. As shown, the structure of the remainder of the stent 310 preferably retains its composition.

In practice, an expandable stent is provided having biodegradable connectors joining a plurality of cylindrical, serpentine ring structures in an unexpanded state. The stent may be self-expanding or balloon expandable. Any of the stent configurations previously discussed may be utilized, amongst others. The stent is delivered in its unexpanded state to a final destination within the body, such as stenosis or area of blockage within a vessel or artery. Generally, this is accomplished by advancing a catheter device through a patient's body in its unexpanded state by deploying a small guide wire and advancing the catheter device over the guide wire to its final destination. The stent is then expanded by inflating a balloon or releasing the self-expanding stent by methods known in the art to expand the stent radially so that the stent abuts against the walls of the artery. Preferably, within the range of thirty (30) days to one-hundred-eighty (180) days after the stent has been expanded within the body, the connector members substantially biodegrade to leave a stent with substantially disjoined ring structures. As discussed, other portions of the stent structure may also substantially biodegrade to allow for a more flexible stent structure.

Stents according to the present invention can be fabricated by any suitable process known in the art. The fabrication process need only be able to produce the ring architecture and biodegradable connector segments of the invention. The stents can be manufactured by having the stent pattern in a rod which would act as a mold. The ring structures of the stent may be set in this mold, and the biodegradable material used to form the connecting members could be injected into the mold to create the biodegradable connecting members. During manufacturing, anti-proliferate compounds may be incorporated into the biodegradable connecting members to inhibit cell proliferation.

Although the present invention has been described with reference to preferred embodiments, those skilled in the art will recognize that changes may be made in form and detail without departing from the scope of the invention as defined in the appended claims. As such, it is intended that the foregoing detailed description be regarded as illustrative rather than limiting and that the appended claims are intended to define the scope of the invention.

## Claims

1. An expandable stent (110) comprising:
a plurality of substantially cylindrical, serpentine ring structures (114), wherein each ring structure (114) extends around a circumference of the stent (110) and comprises at least one unit structure, wherein said at least one unit structure comprises a plurality of strut members (126) and a plurality of bends (130), said strut members (126) and bends (130) forming a substantially zig-zag pattern; and
at least one connector member (118) joining two of said ring structures (114) when said stent is in an unexpanded state, wherein said at least one connector member (118) is biodegradable and adapted to biodegrade when said stent is deployed so that two ring structures become substantially disjoined; **characterised in that** the or each connector member is formed of a plurality of layers, wherein one said layer has a rate of degradation different from that of the other layer or layers.

2. An expandable stent according to claim 1, wherein each layer of the or each connector member is biodegradable.

3. An expandable stent according to claim 1, wherein the ring structure (114) includes a non-biodegradable base material.

4. An expandable stent according to claim 1, 2 or 3, wherein said stent (110) is a substantially integral, tubular shape in said expanded state.

5. An expandable stent of claim 1, 2 or 3, wherein said stent (30) is substantially Y-shaped when in said expanded state.

6. An expandable stent according to any preceding claim, wherein said at least one connector member (118) is made of one or more of polymers, copolymers, block polymers, poly-lactic acid, poly-glycolic acid, polyglycolides, polyactides, polycaprolactones, polyglycerol sebacate, polycarbonates, polyethylene oxide, polybutylene terepthalate, polydioxanones, hybrids, composites, collagen matrices with growth modulators, proteoglycans, glycosaminoglycans, vacuum formed small intestinal submucosa, fibres, chitin, and dextran.

7. An expandable stent according to any preceding claim, wherein said at least one connector member (118) is adapted to biodegrade within thirty days to one-hundred eighty days after said stent is deployed.

8. An expandable stent according to any preceding claim, wherein said ring structures (114) are made of one or more of nitinol, stainless steel, 316 L stainless steep, cobalt chromium, nickel titanium, platinum, and inconel.

9. An expandable stent according to any preceding claim, wherein said ring structures (114) comprise a non-biodegradable base material and one or more biodegradable coating layers.

10. An expandable stent according to claim 9, wherein said one or more biodegradable coating layers are adapted to biodegrade within thirty days to one-hundred eighty days after said stent is deployed.

11. An expandable stent according to claim 9 or 10, wherein said ring structures (114) comprise multiple biodegradable coating layers having varying degradation rates.

12. An expandable stent according to claim 9 or 10, wherein said ring structures (114) comprise one biodegradable coating layer having a uniform degradation rate.

13. An expandable stent according to any one of claims 9 to 12, wherein said ring structures (114) comprise a base material made of a combination of non-biodegradable materials and biodegradable polymers.

14. An expandable stent according to claim 13, wherein said biodegradable polymers of said base material are adapted to biodegrade within thirty days to one-hundred eighty days after said stent is deployed.

15. An expandable stet according to claim 13 or 14, wherein said biodegradable polymers of said base material have varying degradation rates and are disposed unevenly throughout said base material.

16. An expandable stent according to claim 13 or 14, wherein said biodegradable polymers of said base material are disposed uniformly throughout said base material to provide a uniform degradation rate.

17. An expandable stent accordingly any preceding claim, wherein said stent (110) is one of self-expanding and balloon-expandable.

18. An expandable stent according to claim 1, wherein when said at least one connector member (118) biodegrades said two ring structures (114) become completely disjoined so that said stent does not form an integral structure.

19. An expandable stent according to any preceding claim, wherein said at least one connector member (118) is flexible prior to the stent (110) being expanded.

20. An expandable stent according to any preceding claim, wherein said at least one connector member (118) is substantially U or V shaped, is curved or is straight.

21. An expandable stent according to any preceding claim, wherein when said stent (110) is in an unexpanded state said at least one connector member (118) has first and second ends, said first end being connected to one of said plurality of bends (130) of one of said two ring structures (114) and said second end being connected to another of said plurality of bends (130) of the other of said two ring structures (114).

22. An expandable stent according to any preceding claim, wherein when said stent is in an unexpanded state there are two or more connector members (118) joining said two ring structures (114) and adjacent connector members (118) are circumferentially aligned.

23. An expandable stent according to any preceding claim, wherein adjacent ring structures (114) are axially aligned.

24. An expandable stent according to any preceding claim, wherein a plurality of substantially straight tie-bars (138) join a plurality of said ring structures (114).

## Patentansprüche

1. Expandierbarer Stent (110), der Folgendes umfasst:
eine Vielzahl von im Wesentlichen zylindrischen serpentinenförmigen Ringkonstruktionen (114), worin sich jede Ringkonstruktion (114) um einen Umfang des Stents (110) erstreckt und zumindest eine Einheitskonstruktion umfasst, worin die zumindest eine Einheitskonstruktion eine Vielzahl von Strebenelementen (126) und eine Vielzahl von Biegungen (130) umfasst, wobei die Strebenelemente (126) und die Biegungen (130) im Wesentlichen ein Zickzackmuster bilden; und
zumindest ein Verbindungselement (118), das die zwei der Ringkonstruktionen (114) miteinander verbindet, wenn sich der Stent in einem nicht expandierten Zustand befindet, worin das zumindest eine Verbindungselement (118) biologisch abbaubar ist und biologisch abgebaut wird, wenn der Stent abgelegt wird, so dass zwei Ringkonstruktionen im Wesentlichen getrennt werden;
**dadurch gekennzeichnet, dass** das oder jedes Verbindungselement aus einer Vielzahl von Schichten geformt ist, worin eine solche Schicht eine Abbaurate aufweist, die sich von der Abbaurate der anderen Schicht oder der anderen Schichten unterscheidet.

2. Expandierbarer Stent nach Anspruch 1, worin jede Schicht des oder jedes Verbindungselements biologisch abbaubar ist.

3. Expandierbarer Stent nach Anspruch 1, worin die Ringkonstruktion (114) ein nicht biologisch abbaubares Grundmaterial aufweist.

4. Expandierbarer Stent nach Anspruch 1, 2 oder 3, worin der Stent (110) im expandierten Zustand eine im Wesentlichen integrale röhrenförmige Gestalt aufweist.

5. Expandierbarer Stent nach Anspruch 1, 2 oder 3, worin der Stent (30) im expandierten Zustand im Wesentlichen Y-förmig ist.

6. Expandierbarer Stent nach einem der vorhergehenden Ansprüche, worin das zumindest eine Verbindungselement (118) aus ein oder mehr der folgenden Materialien besteht: Polymere, Copolymere, Blockpolymere, Polymilchsäure, Polyglykolsäure, Polyglykolide, Polyactide, Polycaprolactone, Polyglycerolsebacat, Polycarbonate, Polyethylenoxid, Polybutylenterephthalat, Polydioxanone, Hybride, Komposite, Kollagenmatrizen mit Wachstumsmodulatoren, Proteoglykane, Glykosaminoglykane, in Vakuum geformte Dünndarmsubmukosa, Fasern, Chitin und Dextran.

7. Expandierbarer Stent nach einem der vorhergehenden Ansprüche, worin das zumindest eine Verbindungselement (118) innerhalb von dreißig Tagen bis einhundertachtzig Tagen nach Ablage des Stents biologisch abgebaut wird.

8. Expandierbarer Stent nach einem der vorhergehenden Ansprüche, worin die Ringkonstruktionen (114) aus ein oder mehr der folgenden Materialien bestehen: Nitinol, Edelstahl, 316 L Edelstahl, Kobaltchrom, Nickeltitan, Platin und Inconel.

9. Expandierbarer Stent nach einem der vorhergehenden Ansprüche, worin die Ringkonstruktionen (114) ein nicht biologisch abbaubares Grundmaterial und ein oder mehr biologisch abbaubare Überzugsschichten umfassen.

10. Expandierbarer Stent nach Anspruch 9, worin die ein oder mehr biologisch abbaubaren Überzugsschichten innerhalb von dreißig Tagen bis einhundertachtzig Tagen nach Ablage des Stents biologisch abgebaut wird.

11. Expandierbarer Stent nach Anspruch 9 oder 10, worin die Ringkonstruktionen (114) mehrere biologisch abbaubare Überzugsschichten mit unterschiedlichen Abbauraten umfassen.

12. Expandierbarer Stent nach Anspruch 9 oder 10, worin die Ringkonstruktionen (114) eine biologisch abbaubare Überzugsschicht mit einer gleichmäßigen Abbaurate umfassen.

13. Expandierbarer Stent nach einem der Ansprüche 9 bis 12, worin die Ringkonstruktionen (114) ein Grundmaterial aus einer Kombination von nicht biologisch abbaubaren Materialien und biologisch abbaubaren Polymeren umfassen.

14. Expandierbarer Stent nach Anspruch 13, worin die biologisch abbaubaren Polymere des Grundmaterials innerhalb von dreißig Tagen bis einhundertachtzig Tagen nach Ablage des Stents biologisch abgebaut werden.

15. Expandierbarer Stent nach Anspruch 13 oder 14, worin die biologisch abbaubaren Polymere des Grundmaterials unterschiedliche Abbauraten aufweisen und ungleichmäßig durch das Grundmaterial angeordnet sind.

16. Expandierbarer Stent nach Anspruch 13 oder 14, worin die biologisch abbaubaren Polymere des Grundmaterials gleichmäßig im gesamten Grundmaterial angeordnet sind, um eine gleichmäßige Abbaurate zu ergeben.

17. Expandierbarer Stent nach einem der vorhergehenden Ansprüche, worin der Stent (110) selbstexpandierend oder ballon-expandierbar ist.

18. Expandierbarer Stent nach Anspruch 1, worin sich die beiden Ringkonstruktionen (114) vollständig voneinander trennen, wenn das zumindest eine Verbindungselement (118) biologisch abgebaut wird, so dass der Stent keine integrale Konstruktion bildet.

19. Expandierbarer Stent nach einem der vorhergehenden Ansprüche, worin das zumindest eine Verbindungselement (118) flexibel ist, bevor der Stent (110) expandiert wird.

20. Expandierbarer Stent nach einem der vorhergehenden Ansprüche, worin das zumindest eine Verbindungselement (118) im Wesentlichen U- oder V-förmig, gebogen oder gerade ist.

21. Expandierbarer Stent nach einem der vorhergehenden Ansprüche, worin das zumindest eine Verbindungselement (118) erste und zweite Enden aufweist, wenn sich der Stent (110) im nicht expandierten Zustand befindet, wobei das erste Ende mit einer der Vielzahl von Biegungen (130) von einer der beiden Ringkonstruktionen (114) verbunden ist und das zweite Ende mit einer anderen der Vielzahl von Biegungen (130) der anderen der beiden Ringkonstruktionen (114) verbunden ist.

22. Expandierbarer Stent nach einem der vorhergehenden Ansprüche, worin zwei oder mehr Verbindungselemente (118) die beiden Ringkonstruktionen (114) verbinden, wenn sich der Stent in einem nicht expandierten Zustand befindet, und benachbarte Verbindungselemente (118) um den Umfang ausgerichtet sind.

23. Expandierbarer Stent nach einem der vorhergehenden Ansprüche, worin benachbarte Ringkonstruktionen (114) axial ausgerichtet sind.

24. Expandierbarer Stent nach einem der vorhergehenden Ansprüche, worin eine Vielzahl von im Wesentlichen geraden Zugstange (138) eine Vielzahl der Ringkonstruktionen (114) verbinden.

## Revendications

1. Stent (110) expansible comportent :
une pluralité de structures (114) annulaires en serpentin sensiblement cylindriques, dans lequel chaque structure (114) annulaire s'étend autour d'une circonférence du stent (110) et comporte au moins une structure unitaire, dans lequel ladite au moins une structure unitaire comporte une pluralité d'éléments (126) de renfort et une pluralité d'éléments courbes (130), lesdits éléments (126) de renfort et éléments courbes (130) formant un motif sensiblement en zigzag ; et
au moins un élément (118) de liaison joignant deux desdites structures (114) annulaires lorsque ledit stent est dans un état non dilaté, dans lequel ledit au moins un élément (118) de liaison est biodégradable et conçu pour se biodégrader lorsque ledit stent est déployé de sorte que deux structures annulaires deviennent sensiblement disjointes ; **caractérisé en ce que** ledit ou chaque élément de liaison est formé d'une pluralité de couches, dans lequel une desdites couches a une vitesse de dégradation différente de celle de l'autre ou des autres couches.

2. Stent expansible selon la revendication 1, dans lequel chaque couche dudit ou de chaque élément de liaison est biodégradable.

3. Stent expansible selon la revendication 1, dans lequel la structure (114) annulaire comprend un matériau de base non biodégradable.

4. Stent expansible selon la revendication 1, 2 ou 3, dans lequel ledit stent (110) a une forme sensiblement intégralement tubulaire dans ledit état dilaté.

5. Stent expansible selon la revendication 1, 2 ou 3, dans lequel ledit stent (30) a une forme sensiblement en Y lorsque dans ledit état dilaté.

6. Stent expansible selon l'une quelconque des revendications précédentes, dans lequel ledit au moins un élément (118) de liaison est constitué d'un ou plusieurs composants suivants : des polymères, des copolymères, des polymères blocs, de l'acide polylactique, de l'acide polyglycolique, des polyglycolides, des polyactides, des polycaprolactones, du sébacate de polyglycérol, des polycarbonates, de l'oxyde de polyéthylène, du téréphtalate de polybutylène, des polydioxanones, des hybrides, des composites, des matrices de collagène avec modulateurs de croissance, des protéoglycanes, des glycosaminoglycanes, de la sous-muqueuse d'intestin grêle formée sous vide, des fibres, de la chitine et du dextrane.

7. Stent expansible selon l'une quelconque des revendications précédentes, dans lequel ledit au moins un élément (118) de liaison est conçu pour se biodégrader sur une période de 30 à 180 jours après le déploiement dudit stent.

8. Stent expansible selon l'une quelconque des revendications précédentes, dans lequel lesdites structures (114) annulaires sont fabriquées en un ou plusieurs des matériaux suivants : du nitinol, de l'acier inoxydable, de l'acier inoxydable 316 L, du cobalt chrome, du nickel-titane, du platine, et de l'inconel.

9. Stent expansible selon l'une quelconque des revendications précédentes, dans lequel lesdites structures (114) annulaires comportent un matériau de base non biodégradable et une ou plusieurs couches de revêtement biodégradable.

10. Stent expansible selon la revendication 9, dans lequel lesdites une ou plusieurs couches de revêtement biodégradable sont conçues pour se biodégrader sur une période de 30 à 180 jours après le déploiement dudit stent.

11. Stent expansible selon la revendication 9 ou 10, dans lequel lesdites structures (114) annulaires comportent des couches de revêtement biodégradable multiples ayant des vitesses de dégradation variées.

12. Stent expansible selon la revendication 9 ou 10, dans lequel lesdites structures (114) annulaires comportent une couche de revêtement biodégradable ayant une vitesse de dégradation uniforme.

13. Stent expansible selon l'une quelconque des revendications 9 à 12, dans lequel lesdites structures (114) annulaires comportent un matériau de base fabriqué dans une combinaison de matériaux biodégradables et de polymères biodégradables.

14. Stent expansible selon la revendication 13, dans lequel lesdits polymères biodégradables dudit matériau de base sont conçus pour se biodégrader sur une période de 30 à 180 jours après le déploiement dudit stent.

15. Stent expansible selon la revendication 13 ou 14, dans lequel lesdits polymères biodégradables dudit matériau de base ont des vitesse de dégradation variées et sont agencés non uniformément dans l'ensemble du dudit matériau de base.

16. Stent expansible selon la revendication 13 ou 14, dans lequel lesdits polymères biodégradables dudit matériau de base sont agencés uniformément à travers ledit matériau de base pour que la vitesse de dégradation soit uniforme.

17. Stent expansible selon l'une quelconque des revendications précédentes, dans lequel ledit stent (110) est auto-expansible et/ou expansible par ballonnet.

18. Stent expansible selon la revendication 1, dans lequel lorsque ledit au moins un élément (118) de liaison se biodégrade lesdites deux structures (114) annulaires deviennent complètement disjointes de sorte que ledit stent ne forme pas une structure intégrale.

19. Stent expansible selon l'une quelconque des revendications précédentes, dans lequel ledit au moins un élément (118) de liaison est souple avant que le stent (110) soit dilaté.

20. Stent expansible selon l'une quelconque des revendications précédentes, dans lequel ledit au moins un élément (118) de liaison a une forme sensiblement en U ou V, est courbe ou est rectiligne.

21. Stent expansible selon l'une quelconque des revendications précédentes, dans lequel lorsque ledit stent (110) est dans un état non dilaté ledit au moins un élément (118) de liaison présente une première et une seconde extrémités, ladite première extrémité étant reliée à un élément courbe de ladite pluralité d'éléments courbes (130) d'une desdites deux structures (114) annulaires et ladite seconde extrémité est reliée à un autre élément courbe de ladite pluralité d'éléments courbes (130) de l'autre desdites deux structures (114) annulaires.

22. Stent expansible selon l'une quelconque des revendications précédentes, dans lequel lorsque ledit stent est dans un état non dilaté, deux ou plusieurs éléments (118) de liaison joignant lesdites deux structures (114) annulaires et les éléments (118) de liaison adjacents sont alignés de manière circonférentielle.

23. Stent expansible selon l'une quelconque des revendications précédentes dans lequel les structures (114) annulaires sont alignées axialement.

24. Stent expansible selon l'une quelconque des revendications précédentes, dans lequel une pluralité de barres de raccordement (138) sensiblement rectilignes joignent une pluralité desdites structures (114) annulaires.
